Europäisches Patentamt

European Patent Office

Office européen des brevets

(11) Veröffentlichungsnummer: **0 255 899 B1**

(19)

(12) **EUROPÄISCHE PATENTSCHRIFT**

(45) Veröffentlichungstag der Patentschrift: **15.07.92**

(21) Anmeldenummer: **87110713.2**

(22) Anmeldetag: **24.07.87**

(51) Int. Cl.⁵: **A61N 1/365**, A61N 1/368

(54) **Frequenzadaptierender Herzschrittmacher.**

(30) Priorität: **31.07.86 DE 3625894**

(43) Veröffentlichungstag der Anmeldung:
**17.02.88 Patentblatt 88/07**

(45) Bekanntmachung des Hinweises auf die
Patenterteilung:
**15.07.92 Patentblatt 92/29**

(84) Benannte Vertragsstaaten:
**DE FR GB IT NL SE**

(56) Entgegenhaltungen:
**EP-A- 0 194 224**
**WO-A-86/05698**
**GB-A- 2 079 610**
**US-A- 4 108 148**
**US-A- 4 228 803**

(73) Patentinhaber: **Irnich, Werner, Prof. Dr.-Ing.**
**Birkenweg 60**
**W-6301 Wettenberg 3(DE)**

(72) Erfinder: **Irnich, Werner, Prof. Dr.-Ing.**
**Birkenweg 60**
**W-6301 Wettenberg 3(DE)**

(74) Vertreter: **Biermann, Wilhelm, Dr.-Ing.**
**Morillenhang 39**
**W-5100 Aachen(DE)**

# Beschreibung

Der Erfindung betrifft einen frequenzadaptierenden Herzschrittmacher.

Frequenzadaptierende Herzschrittmacher haben die Aufgabe, die Frequenz der Stimulationsimpulse den jeweiligen Bedürfnissen des Kreislaufs anzupassen. Derartige frequenzadaptierende Herzschrittmacher arbeiten im Ruhezustand des Körpers mit niedriger Frequenz und erhöhen ihre Frequenz entsprechend der körperlichen Beanspruchung bis zu einem individuell festlegbaren Höchstwert. Um den jeweiligen Belastungszustand des Körpers bzw. des Kreislaufs festzustellen, benötigen solche Schrittmachersysteme einen Meßfühler, mit dem eine vom Belastungszustand des Kreislaufs abhängige Größe gemessen und in Abhängigkeit von dem Meßwert die Impulsfrequenz geregelt wird.

Bei einem aus der US-A-4.228.803 bekannten frequenzadaptierenden Herzschrittmacher wird der Ventrikel stimuliert, und die im Ventrikel angeordnete Stimulationselektrode dient gleichzeitig als Meßfühler. Dieser Herzschrittmacher macht sich die Tatsache zunutzte, daß sich das Zeitintervall, das sich im EKG zwischen der Q-Zacke und der T-Welle ergibt, mit zunehmender körperlicher Belastung verkürzt. In der Zeitmeßschaltung dieses Herzschrittmachers wird das Zeitintervall zwischen dem Stimulationsimpuls und der darauffolgenden T-Welle gemessen, und in Abhängigkeit von dem gemessenen Zeitintervall wird in der Auswerteschaltung die Folgefrequenz der Stimulationsimpulse gesteuert (Stimulus-T-Prinzip). Dieser bekannte frequenzadaptierende Herzschrittmacher ist nur im Ventrikel anwendbar, nicht dagegen im Atrium, weil im Atrium ein der T-Welle äquivalentes Signal nicht erfaßt wird. Das hat zur Folge, daß sich dieser bekannte Herzschrittmacher für alle die jenigen Patienten, bei denen eine Erregungsbildungs- oder Erregungsleitungskrankheit nur des Atriums vorliegt, und das sind mehr als ein Drittel aller Herzschrittmacher-Patienten, nicht verwenden läßt. Denn der Einsatz dieses bekannten Herzschrittmachers würde bei den genannten Patienten dazu führen, daß eine mögliche Synchronisation zwischen Atrium und Ventrikel unterbleibt. Man muß in diesen Fällen sogar befürchten, daß die Stimulation des Ventrikels zu einer retrograden Atriumerregung führt, die fast immer zu einer Kontraktion des Atriums gegen die geschlossenen Herzklappen führt. Dadurch ergibt sich der physiologische Nachteil, daß vom Atrium das Blut nicht in die Hauptkammern gepumpt, sondern statt dessen in die Venen zurückgepumpt wird.

Auch der aus der US-A-4.527.568 bekannte Herzschrittmacher benutzt das Zeitintervall zwischen dem Stimulationsimpuls und der T-Welle als Steuergröße bei einer Steuerschaltung zur Steuerung der Stimulationsfrequenz. In diesem Fall handelt es sich um ein System, bei dem ein atrialgesteuerter Ventrikel-Schrittmacher mit dem bekannten Stimulus-T-Prinzip kombiniert wird. Darüber hinaus ist noch ein zusätzlicher Sensor vorgesehen, wobei durch diesen zusätzlichen Sensor ein weiterer vom Belastungszustand abhängiger Parameter, wie beispielsweise der pH-Wert, der venöse Sauerstoffgehalt, die Körpertemperatur oder die Atmungsgeschwindigkeit gemessen wird. Dieser bekannte Herzschrittmacher kann auch bei Patienten mit einem AV-Block eingesetzt werden.

Bei einem anderen, aus der US-A-4.108.148 bekannten Herzschrittmacher wird der Ventrikel stimuliert, und das als Steuergröße dienende Signal wird im Atrium wahrgenommen. Hierbei wird als bekannt vorausgesetzt, daß bei funktionierender AV-Überleitung das AV-Intervall ($T_{AR}$-Zeitintervall) nicht konstant ist; sondern sich in Abhängigkeit von der Impulsfrequenz ändert, indem es sich von einem relativ langen Zeitintervall bei niedriger Pulsfrequenz zu einem relativ kurzen Zeitintervall bei hoher Pulsfrequenz verkürzt. Der hier beschriebene Schrittmacher paßt sich diesem natürlichen Verhalten des Herzens an, indem er bei Wahrnehmung einer erhöhten Frequenz der atrialen Eigenaktionen bei der ventrikulären Stimulation diese Verkürzung der AV-Zeit berücksichtigt.

Auch ein anderer, in der US-A-4.060.090 beschriebener Herzschrittmacher setzt als bekannt voraus, daß das AV-Intervall von der Impulsfrequenz abhängig ist und sich mit zunehmender Impulsfrequenz verkürzt, und berücksichtigt diesen Effekt bei der Stimulation des Ventrikels.

Die GB-A-2.079.610 beschreibt einen Herzschrittmacher mit mehreren Stimulationselektroden und einem vielfach programmierbaren Mikroprozessor, wobei die Erzeugung der Stimulationsimpulse nur unter dem Gesichtspunkt des "Demand Sensing" und der Telemetrie erfolgt. Eine selbsttätige Anpassung der Stimulationsfrequenz erfolgt bei diesem Herzschrittmacher nicht.

Zum nicht vorveröffentlichten Stand der Technik gehört die EP-A-0.194.224. Bei dem dort beschriebenen Herzschrittmacher erfolgt eine Steuerung der Stimulationsfrequenz mit Hilfe von aus dem Ventrikel ableitbaren Zeitparametern, die mit Hilfe eines Mikrophons erfaßt werden.

Ebenfalls zum nicht vorveröffentlichten Stand der Technik gemäß Art. 54(3) EPÜ gehört auch die WO-A-86/05698, die gemäß Art. 158 EPÜ an die Stelle der EP-A 0 220 194 tritt. Diese Druckschrift beschreibt einen frequenzadaptierenden Herzschrittmacher mit einer für die Verankerung im Atrium vorgesehenen Stimulationselektrode, einer für die Verankerung im Ventrikel vorgesehenen Überwachungselektrode, einer von dem Impulsgenerator und von der Überwachungselektrode angesteuerten elektronischen Zeitmeßschaltung zur Messung des Zeitintervalls $T_{AR}$ zwischen dem Stimulationsimpuls im Atrium und der von der Überwachungselektrode festgestellten diesem Stimulationsimpuls zugeordneten Erregung im Ventrikel, sowie einer von der Zeitmeßschaltung angesteuerten elektronischen Auswerteschaltung. Durch die Auswerteschaltung wird das Zeitintervall zwischen zwei Stimulationsimpulsen in Abhängigkeit von dem gemessenen Zeitintervall $T_{AR}$ zwischen Stimulationsimpuls und zugeordneter Erregung im Ventrikel geregelt. Bei diesem frequenzadaptierenden Herzschrittmacher erfolgt jedoch die Regelung des Zeitintervalls zwischen zwei Stimulationsimpulsen nicht in Form einer spezifierten Abhängigkeit von dem gemessenen Zeitintervall $T_{AR}$ zwischen dem Stimulationsimpuls und der diesem zugeordneten Erregung im Ventrikel. Zwar geht ein Ausführungsbeispiel von einer begrenzt linearen Funktion innerhalb eines Algorithmus aus, der den Zusammenhang zwischen einem über mehrere aufeinanderfolgende Zeitintervalle $T_{AR}$ gemittelten Wert und der Stimulationsfrequenz beschreibt. Doch folgen im übrigen die in dieser Druckschrift als Ausführungsbeispiele angegebenen Verknüpfungen zwischen dem gemessenen Zeitintervall $T_{AR}$ und der Stimulationsfrequenz $F_{St}$ anderen Gesetzen.

Der Erfindung liegt die Aufgabe zugrunde, einen die Stimulationsfrequenz an die jeweilige körperliche Belastung anpassenden Herzschrittmacher zu schaffen, der von einer gut und mit verhältnismäßig einfachen Mitteln erfaßbaren Meßgröße ausgeht, eine eindeutige Abhängigkeit der Stimulationsfrequenz von der erfaßten Meßgröße angibt, und der für die Stimulation des Atriums bei solchen Patienten einsetzbar ist, bei denen ein Erregungsbildungs- oder ein Erregungsleitungsdefekt in Atriumebene vorliegt, bei denen jedoch die sogenannte Atrium-Ventrikel-Überleitung noch intakt ist.

Diese Aufgabe wird durch einen Herzschrittmacher mit den im Patentanspruch 1 angegebenen Merkmalen gelöst.

Es ist zwar bekannt, daß sich bei einer natürlichen Frequenzerhöhung, beispielsweise als Folge einer erhöhten körperlichen Belastung, die AV-Überleitungszeit verkürzt. Jedoch war es nicht bekannt und nicht zu erwarten, daß die AV-Überleitungszeit sich auch dann verkürzt, wenn sich die körperliche Belastung erhöht, ohne daß sich dabei die Schlagfrequenz des Herzens erhöht, das heißt die erhöhte Herzleistung im wesentlichen durch Vergrößerung des Schlagvolumens erfolgt. Die Erfindung macht sich diese neue Erkenntnis zunutze, daß auch die Erhöhung der körperlichen Belastung allein ohne eine gleichzeitige Erhöhung der Schlagfrequenz zu einer deutlich meßbaren und zum Grad der Belastungserhöhung proportionalen Verkürzung der AV-Zeit führt. Auf dieser Erkenntnis aufbauend wird erfindungsgemäß ein frequenzadaptierender Herzschrittmacher vorgeschlagen, der eine auf verhältnismäßig einfache und sichere Weise festzustellende Meßgröße als Parameter für den jeweiligen körperlichen Belastungszustand für die Steuerung der Stimulationsfrequenz benutzt. Berücksichtigt man, daß eine Verkürzung der AV-Zeit um 10 Millisekunden eine zusätzliche körperliche Belastung repräsentiert, die eine Erhöhung der Schlagfrequenz um etwa 10 pro Minute erfordert, dann erkennt man, daß es sich hierbei um zeitliche Meßgrößen handelt, die mit üblichen elektronischen Mitteln gut meßbar und auswertbar sind.

In weiterer vorteilhafter Ausgestaltung der Erfindung kann die elektronische Auswerteschaltung des Herzschrittmachers so ausgebildet sein, daß die Stimulationsfrequenz auf die dem jeweiligen Belastungsgrad zugeordnete optimale Frequenz geregelt wird. Bei dieser Regelungsschaltung macht die Erfindung sich die Entdeckung zunutze, daß bei Stimulation des Atriums mit unphysiologisch hohen Frequenzen sich die AV-Zeit überraschenderweise wieder verlängert und daß infolgedessen jedem körperlichen Belastungsgrad ein Minimum im Verlauf der AV-Zeit zugeordnet ist, wobei dieses Minimum der AV-Zeit der jeweils optimalen Schlagfrequenz entspricht. Zur Ausnutzung dieses Effektes kann also zusätzlich eine Auswerteschaltung vorgesehen sein, in der bei zunehmender Stimulationsfrequenz die Änderung der AV-Zeit gemessen und bei Feststellung einer Verlängerung der AV-Zeit die Stimulationsfrequenz wieder verringert wird. Auf diese Weise wird die Stimulationsfrequenz jeweils auf einen optimalen Wert geregelt, der der jeweils minimalen AV-Zeit entspricht.

Bevorzugte Ausführungsformen des erfindungsgemäßen Herzschrittmachers werden nachfolgend anhand der Zeichnungen näher beschrieben.

Von den Zeichnungen zeigt

Fig.1 den grundsätzlichen Aufbau eines erfindungsgemäßen Herzschrittmachers in schematischer Darstellung;

Fig.2 ein Diagramm, das die zeitliche Aufeinanderfolge der Erregung im Atrium und im Ventrikel veranschaulicht;

Fig.3 ein Blockschaltbild für die Ausbildung der Steuerschaltung in Analogtechnik;

Fig.4 ein Blockschaltbild für eine in Digitaltechnik ausgebildete Steuerschaltung;

Fig 5 ein Blockschaltbild für eine Steuerschaltung, die zusätzlich auf Spontanaktionen des Atriums reagiert;

Fig 6 ein Diagramm, das die Wirkungsweise des in Fig 5 dargestellten Herzschrittmachers veranschaulicht;

Fig 7    ein Diagramm, das den Zusammenhang zwischen der $T_{AR}$-Zeit und der
Stimulationsfrequenz bei verschiedenen Belastungszuständen darstellt; und

Fig 8    ein Flußdiagramm für die Suche der
optimalen Stimulationsfrequenz.

Der erfindungsgemäße Herzschrittmacher arbeitet mit zwei im Herzen 1 zu verankernden Elektroden, nämlich der im Atrium 2 zu verankernden
atrialen Stimulationselektrode 4 und der im Ventrikel zu verankernden ventrikulären Überwachungselektrode 6. Die Stimulationselektrode 4 erhält ihre
Stimulationsimpulse von der Schrittmacherschaltung 8 über die Leitung 5. Die Überwachungselektrode 6 gibt die von ihr aufgenommenen elektrischen Signale über die Leitung 7 an die Schrittmacherschaltung 8 weiter.

Die Schrittmacherschaltung 8 umfaßt einen Impulsgenerator 10, der die Stimulationsimpulse einerseits über die Leitung 5 an die Stimulationselektrode 4, und andererseits über die Leitungen 11
und 12 an eine Zeitmeßschaltung 13 abgibt. In der
Zeitmeßschaltung 13 wird mit dem Eintreffen des
über die Leitung 12 am START-Eingang ankommenden Stimulationsimpulses der Meßvorgang für
die Messung des für die Steuerung benutzten Zeitintervalls gestartet. Das von der ventrikulären Überwachungselektrode 6 erfaßte auf den Stimulationsimpuls zurückzuführende Erregungssignal im Ventrikel wird über die Leitung 7 einem ventrikulären
Verstärker 14 zugeführt, der das Signal verstärkt
und in ein für die nachfolgende Zeitmeßschaltung
13 verarbeitbares Signal umformt. Sobald das von
dem Verstärker 14 kommende Signal über die Leitung 15 die Zeitmeßschaltung 13 über den STOP-
Eingang erreicht, wird der Zeitmeßvorgang abgebrochen, und über die Leitung 16 ein Signal zur
Verfügung gestellt, das zu der Atrium-Ventrikel-
Überleitungszeit (AV-Zeit oder $T_{AR}$-Zeit) direkt proportional ist.

Über die Leitung 16 wird eine Auswerteschaltung 18 angesteuert. Die Aufgabe deser Auswerteschaltung 18 besteht darin, entsprechend dem eingehenden Signal das Zeitintervall zwischen der Erregung im Ventrikel und dem nächsten Stimulationsimpuls zu verkürzen bzw. zu verlängern, das
heißt die Stimulationsfrequenz zu erhöhen bzw. zu
erniedrigen.

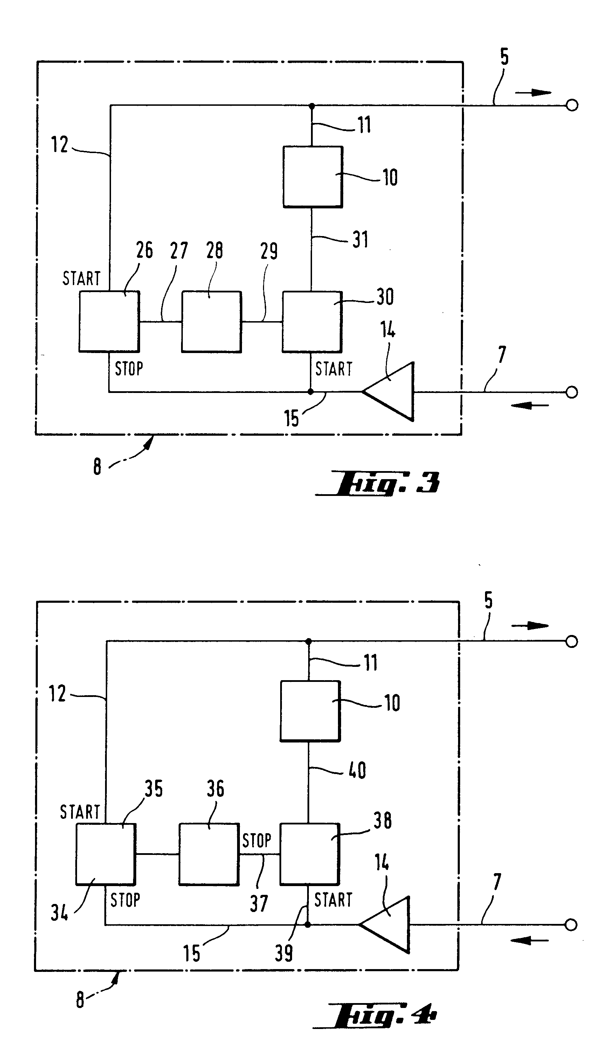

In dem Diagramm in Fig 2 ist die Aufeinanderfolge der Signale im Atrium und im Ventrikel dargestellt. Bezeichnet man das Zeitintervall zwischen
dem atrialen Stimulationsimpuls A des stimulierenden Signals S und der R-Zacke des diesem Stimulationsimpuls A zugeordneten ventrikulären Erregungssignals V mit $T_{AR}$, und das Zeitintervall zwischen der R-Zacke des ventrikulären Erregungssignals V und dem nächstfolgenden Stimulationsimpuls A mit $T_{RA}$, so beträgt die Periodendauer zwischen zwei Stimulationsimpulsen gleich $T_{AR}$ +
$T_{RA}$. Die Stimulationsfrequenz beträgt dementsprechend

$$\frac{1}{T_{AR} + T_{RA}}.$$

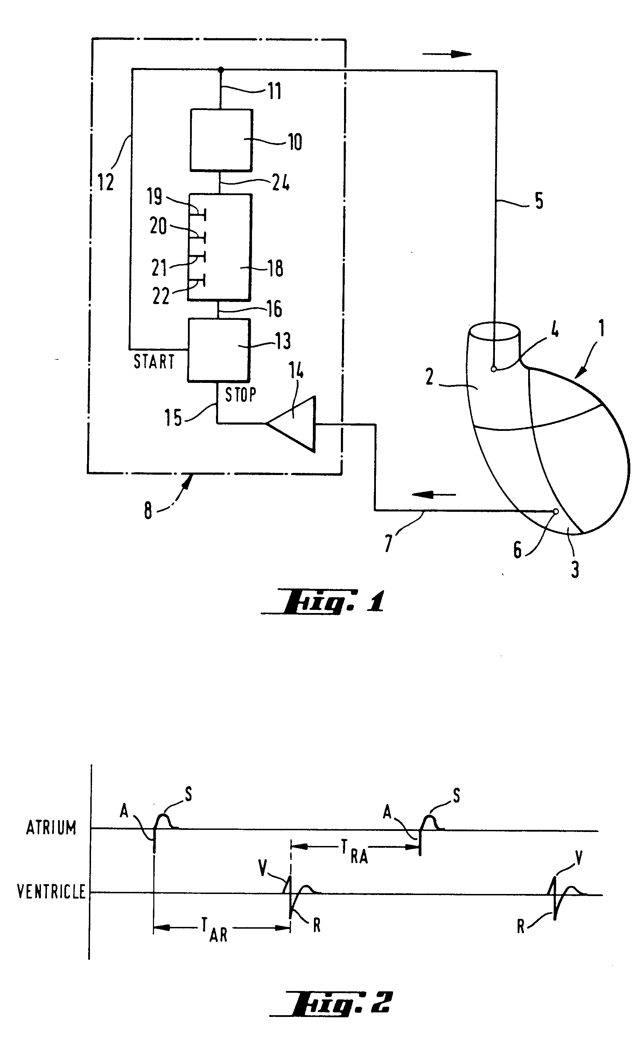

In der Auswerteschaltung 18 (Fig 1) wird nun
die Stimulationsfrequenz $F_{St}$ entsprechend einer
gewählten Funktion errechnet. Beispielsweise wird
eine lineare Verknüpfung zwischen dem $T_{AR}$-Zeitintervall und der Stimulationsfrequenz $F_{St}$ entsprechend der Funktion $F_{St} = -a \cdot T_{AR} + b$ gewählt.
Dabei empfiehlt es sich, die beiden Koeffizienten a
und b patientenindividuell einzustellen. Hierfür sind
in der Auswerteschaltung 18 ein stetig verstellbares
Einstellglied 19 für den Koeffizienten a und ein
Einstellglied 20 für den Koeffizienten b vorgesehen.
Ferner ist ein Einstellglied 21 vorgesehen, mit dem
die Maximalfrequenz festgelegt wird, und ein Einstellglied 22, mit dem die Minimalfrequenz eingestellt wird. Anstelle einer linearen Verknüpfung zwischen der Stimulationsfrequenz und der $T_{AR}$-Zeit
kann jedoch auch eine S-förmige Verknüpfung gewählt werden.

Von der Auswerteschaltung 18 wird über die
Leitung 24 der Impulsgenerator 10 angesteuert, der
in den von dem Funktionsgenerator vorgegebenen
Zeitpunkten die Stimulationssignale erzeugt und an
die Stimulationselektrode 4 abgibt.

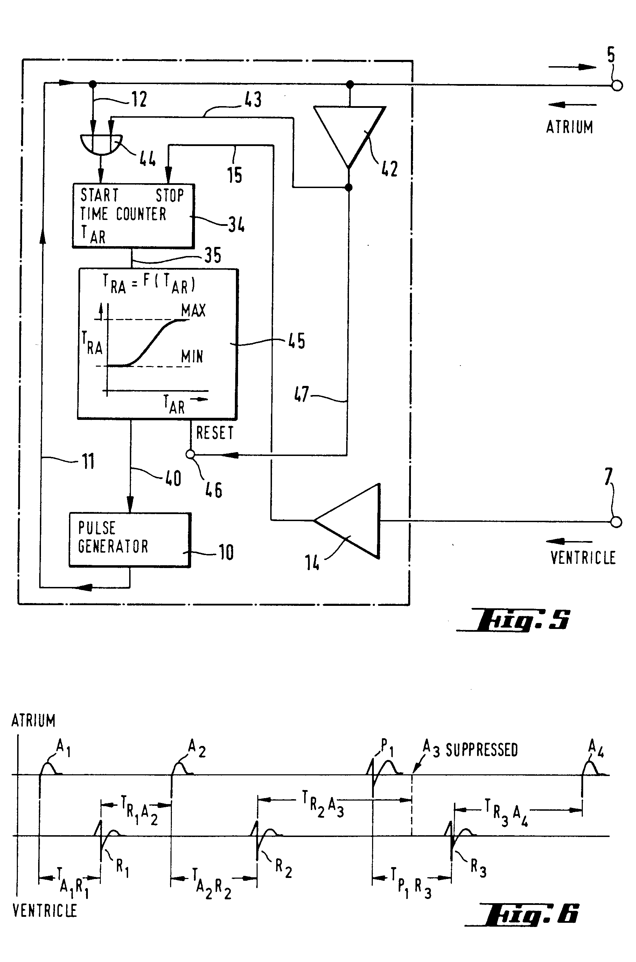

In der Fig 3 ist eine Herzschrittmacherschaltung dargestellt, die nach dem anhand der Fig 1
beschriebenen Prinzip arbeitet und bei der die
Schaltung in Analogtechnik ausgeführt ist. Die Messung des Zeitintervalls $T_{AR}$ erfolgt in der Weise,
daß der Ausgang des Impulsgenerators 10 über die
Leitungen 11 und 12 mit dem START-Eingang eines Spannungsintegrators 26 verbunden ist, während das über die Leitung 7 ankommende Signal
der Überwachungselektrode im ventrikulären Verstärker 14 verstärkt und aufbereitet und über die
Leitung 15 dem STOP-Eingang des Spannungsintegrators 26 zugeführt wird. Der Spannungsintegrator 26 erzeugt eine dem Zeitintervall $T_{AR}$ proportionale Spannung, die über die Leitung 27 einem
analogen Funktionsgenerator 28 zugeführt wird.
Der analoge Funktionsgenerator 28 hat die Aufgabe, aufgrund der eingehenden Spannung die Zeitspanne $T_{RA}$ zu berechnen und das spannungsgesteuerte Monoflop 30 über die Leitung 29 anzusteuern. Das spannungsgesteuerte Monoflop 30,
das außerdem über seinen START-Eingang von
dem ventrikulären Verstärker 14 angesteuert wird,
liefert seinerseits das in Abhängigkeit von der gemessenen AV-Überleitungszeit geregelte Zeitintervall $T_{RA}$ und steuert über die Leitung 31 den Impulsgenerator 10 an.

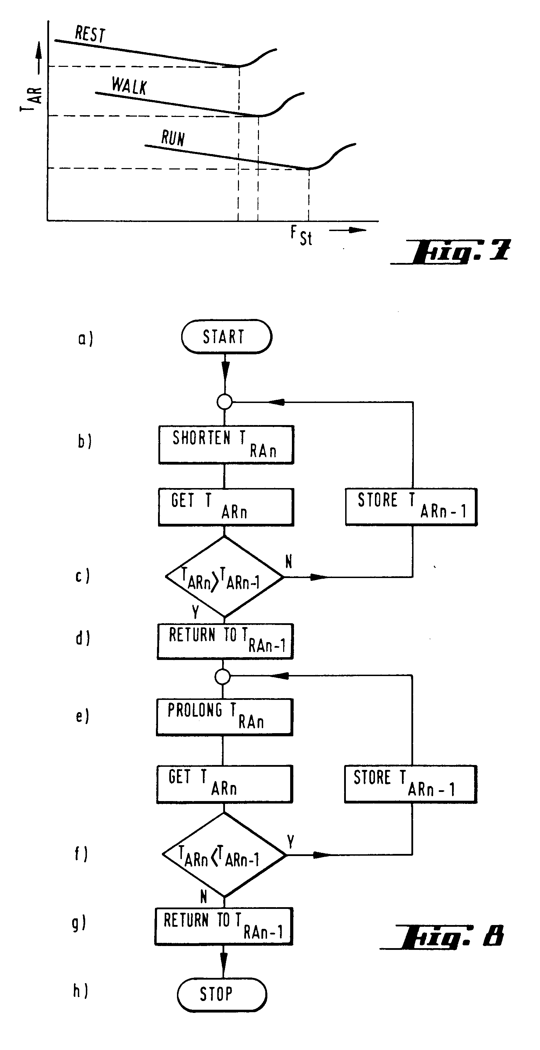

Eine digital arbeitende Ausführungsform des erfindungsgemäßen Schrittmachers ist in Fig 4 als Blockdiagramm veranschaulicht. In diesem Fall wird als Zeitmeßschaltung ein Zähler 34 eingesetzt, dessen START-Eingang über die Leitungen 11, 12 mit dem Impulsgenerator 10 verbunden ist, und der von dem Stimulationsimpuls in Gang gesetzt wird. Der Zählvorgang wird abgebrochen durch das von dem ventrikulären Verstärker 14 über die Leitung 15 am STOP-Eingang des Zählers 34 ankommende Signal. Der Zähler 34 liefert ein dem Zeitintervall $T_{AR}$ entsprechendes digitales Signal über die Leitung 35 an den digitalen Funktionsgenerator 36, der seinerseits aus dem eingehenden digitalen Signal die diesem entsprechende $T_{RA}$-Zeit errechnet. Von dem Funktionsgenerator 36 wird über die Leitung 37 der STOP-Eingang eines Zählers 38 angesteuert. Der START-Eingang dieses Zählers 38 liegt über die Leitung 39 an dem Ausgang des Verstärkers 14. Auf diese Weise wird durch die R-Zacke des ventrikulären Signals der Zähler 38 gestartet, während der Zählvorgang nach dem von dem Funktionsgenerator 36 berechneten Zeitpunkt von diesem abgebrochen wird. Der Zähler 38 steuert über die Leitung 40 den Impulsgenerator 10 an und sorgt dafür, daß die Stimulationsimpulse in den berechneten Zeitpunkten, das heißt nach dem ermittelten Zeitintervall $T_{RA}$ und mit gesteuerter bzw. geregelter Impulsfrequenz, an die Stimulationselektrode abgegeben werden.

Fig 5 zeigt, ebenfalls als Blockschaltbild, eine Ausführungsform des erfindungsgemäßen Herzschrittmachers, die eine zusätzliche Funktion aufweist. In diesem Fall dient nämlich die atriale Stimulationselektrode gleichzeitig zur Wahrnehmung von Spontanaktivitäten, aus denen ein zusätzliches Kriterium für physiologische Stimulation abgeleitet wird.

Es ist bekannt, daß bei einer atrialen Extrasystole bzw. auch bei Tachykardie sich die AV-Überleitungszeit ($T_{AR}$) nicht verkürzt, sondern sich unter Umständen sogar verlängert bis zum totalen Block. Aus der Kenntnis des Auftretens eines Spontan-Signals im Atrium und der sich daran anschließenden AV-Überleitungszeit kann daher abgeleitet werden, ob es sich um eine spontane physiologische Frequenzerhöhung oder um eine pathologische Tachykardie handelt. Wenn also durch die atriale Stimulationselektrode auch Spontanaktionen festgestellt werden, lassen sich nicht nur die Möglichkeiten des Schrittmachers in der Weise erweitern, daß er Tachykardien erkennt, sondern man vermeidet dadurch auch, daß unsynchronisierte Stimulation in atriale Spontanaktionen hinein zu atrialen Tachykardien oder gar zu Vorhofflimmern führt. Diese zweifache Wahrnehmungsfunktion sowohl im Atrium als auch im Ventrikel führt zum einen zu einer Triggerung des Atriums durch den Ventrikel, zum anderen zu Inhibition des atrialen Stimulus durch das Atrium.

Die in Fig 5 dargestellte Ausführungsform des erfindungsgemäßen Herzschrittmachers erfüllt die genannten Funktionen. Grundsätzlich ist sie ähnlich aufgebaut wie die anhand der Fig 3 und 4 beschriebenen Schaltungen, das heißt sie umfaßt ebenfalls eine Zeitmeßschaltung in Form eines Zählers 34, einen Impulsgenerator 10 und eine die Stimulationsfrequenz des Impulsgebers 10 in Abhängigkeit von dem vom Zähler 34 gemessenen Zeitintervall steuernde Auswerteschaltung, bei der es sich in diesem Fall um einen Mikroprozessor 45 handelt. Zusätzlich weist die Schaltung einen atrialen Verstärker 42 und ein ODER-Gatter 44 auf. Das ODER-Gatter 44 hat die Aufgabe, den über die Leitungen 11 und 12 auf den Zähler 34 geschalteten Ausgang des Impulsgebers 10 und den über die Leitung 43 ebenfalls auf den Zähler 34 geschalteten Ausgang des atrialen Verstärkers 42 zu entkoppeln, das heißt elektrisch voneinander zu trennen. Während die Ausgänge des Impulsgebers 10 und des atrialen Verstärkers 42 auf den START-Eingang des Zählers 34 geschaltet sind, ist der STOPP-Eingang des Zählers 34 über die Leitung 15 mit dem Ausgang des ventrikulären Verstärkers 14 verbunden. Das vom Zähler 34 kommende Signal, das der jeweils gemessenen AV-Überleitungszeit $T_{AR}$ entspricht, wird über die Leitung 35 in digitaler Form dem Mikroprozessor 45 zugeführt.

Der Mikroprozessor 45 weist einen RESET-Eingang 46 auf, der über die Leitung 47 mit dem Ausgang des atrialen Verstärkers 42 verbunden ist. Bei Auftreten einer Spontanaktion P wird auf diese Weise einerseits der nächstfolgende Stimulationsimpuls unterdrückt, und andererseits wird durch diese Spontanaktion über die Leitung 43 der Zähler 34 auf Null gesetzt, der nunmehr das mit der Spontanaktion P beginnende Zeitintervall $T_{PR}$ mißt.

Das Wirkungsschema dieser Ausführungsform, soweit es die Berücksichtigung von Spontanaktionen des Herzens betrifft, zeigt Fig 6. In diesem Wirkungsschema sind der zeitliche Ablauf der atrialen Stimulationsimpulse $A_1$, $A_2$ und $A_4$, eine atriale Spontanaktion $P_1$ und die auf $A_1$, $A_2$ und $P_1$ folgenden ventrikulären Erregungen $R_1$, $R_2$ und $R_3$ dargestellt. Insbesondere ist dort zu erkennen, daß bei einer atrialen Spontanaktion $P_1$ der nächstfolgende Stimulationsimpuls $A_3$ unterdrückt und von dieser Spontanaktion $P_1$ ausgehend die Überleitungszeit $T_{P_1R_3}$ gemessen wird.

Der Mikroprozessor 45 ermittelt aufgrund des über die Leitung 35 zugeführten Eingangssignals, das dem gemessenen Zeitintervall $T_{AR}$ entspricht, den jeweils zugehörigen $T_{RA}$-Wert. Die Ermittlung des $T_{RA}$-Wertes erfolgt aufgrund einer dem Mikroprozessor eingespeicherten Tabelle bzw. einer eingespeicherten Funktion, und zwar insbesondere einer linearen Approximation. In einfacher aber wirkungsvoller Weise wird der funktionelle Zusammenhang zwischen $T_{AR}$ und $T_{RA}$ bzw. der Stimulationsfrequenz in der Weise ermittelt, daß bei zwei definierten Leistungszuständen des Patienten die entsprechenden optimalen Frequenzen des Patienten ermittelt und als zwei Wertepaare dem Mikroprozessor 45 eingegeben werden. Der Mikroprozessor 45 ermittelt daraus die lineare Funktion, deren Gültigkeit durch die ebenfalls einprogrammierbare Minimal- und Maximalfrequenz begrenzt wird. Die optimale Frequenz läßt sich dadurch bestimmen, daß man bei einer bestimmten vorgegebenen Leistung die Herzfrequenz durch Probestimulation solange erhöht, bis die $T_{AR}$-Zeit ihr jeweiliges Minimum erreicht hat. Der Zusammenhang zwischen der Stimulationsfrequenz und der $T_{AR}$-Zeit ist in Fig 7 für drei verschiedene Belastungszustände schematisch dargestellt.

Eine weitere Aufgabe des Mikroprozessors besteht darin, bei atrialen Spontanaktionen das $T_{AR}$-Intervall daraufhin zu überprüfen, ob es merklich größer geworden ist als das vorangegangene $T_{AR}$-Zeitintervall. Ist das der Fall, dann wird innerhalb des Mikroprozessors 45 ein antitachykardes Stimulationsprogramm vorbereitet, das nach mehrmaliger Wiederholung vorzeitiger atrialer Spontanaktionen aufgerufen wird und über die Leitung 40 den Impulsgenerator 10 zur antitachykarden Stimulation ansteuert.

Der Mikroprozessor 45 kann ferner so eingerichtet sein, daß er die Stimulationsfrequenz $F_{St}$ auf die dem jeweiligen Belastungsgrad zugeordnete optimale Frequenz regelt. Wie aus Fig 7 hervorgeht, besteht bei jedem Belastungsgrad eine optimale Herzfrequenz, die sich dadurch auszeichnet, daß bei der optimalen Herzfrequenz die zugehörige $T_{AR}$-Zeit jeweils einen minimalen Wert aufweist. Um die Stimulationsfrequenz auf die optimale Frequenz zu regeln, wird der Mikroprozessor 45 so eingerichtet, daß er in periodischen Zeitintervallen prüft, ob die $T_{AR}$-Zeit tatsächlich dem jeweiligen Leistungszustand entspricht. Zu diesem Zweck wird die Stimulationsfrequenz für ein oder mehrere Zeitintervalle erhöht bzw. erniedrigt. Anschließend wird abgefragt, ob das daran anschließende AV-Intervall $T_{AR}$ gleichbleibt oder sich verkürzt oder verlängert. Auf diese Art und Weise wird jeweils das Minimum der $T_{AR}$-Zeit gesucht und so die Stimulationsfrequenz entsprechend diesem Minimum geregelt.

In welcher Weise die Minimumsuche des $T_{AR}$-Intervalls in Abhängigkeit von der Stimulationsfrequenz bzw. in Abhängigkeit vom $T_{RA}$-Intervall erfolgt, ist in dem Flußdiagramm in Fig 8 wiedergegeben. Die in diesem Flußdiagramm dargestellten Entscheidungsschritte bedürfen keiner ausführlichen Erklärung, sondern lassen sich wie folgt kurz beschreiben:

In

a) wird periodisch der Befehl dem Mikroprozessor erteilt, die folgenden Intervalle $T_{AR}$ und $T_{RA}$ abzuspeichern und folgende Maßnahmen bzw. Entscheidungen durchzuführen:

b) Das Intervall $T_{RA}$ soll verkürzt werden.

c) Es wird abgefragt, ob das n-te-Intervall $T_{AR}$ größer als das vorhergehende (n-1)-te Intervall $T_{AR}$ gewesen ist oder nicht. Ist dies nicht der Fall, wird die Schleife nach b) zurückgeführt, um das Intervall $T_{RA}$ weiter zu verkürzen.

d) Ist das n-te Intervall größer als das vorhergehende, wird der Befehl gegeben, auf das vorletzte Intervall zurückzukehren. Damit ist die Abfrage beendet, ob durch Verkürzen des $T_{RA}$-Intervalls eine AV-Zeitverlängerung $T_{AR}$ erfolgte oder nicht.

e) Mit dem Befehl der Verlängerung des $T_{RA}$-Intervalls wird in gleicher Weise wie in der Schleife b)-c) abgefragt, ob das letzte $T_{AR}$-Intervall kleiner als das vorletzte ist.

f) Es wird abgefragt, ob das n-te Intervall $T_{AR}$ kleiner als das vorhergehende (n-1)-te Intervall $T_{AR}$ gewesen ist oder nicht. Ist dies der Fall, wird die Schleife nach e) zurückgeführt.

g) Im Falle der Verneinung wird wieder auf das vorletzte $T_{RA}$-Intervall zurückgegangen.

h) Die Routine zur Kontrolle des optimalen $T_{RA}$-Intervalls ist abgeschlossen.

## Patentansprüche

1. Frequenzadaptierender Herzschrittmacher mit folgenden Merkmalen:
   - einem Impulsgenerator (10);
   - einer für die Verankerung im Atrium vorgesehenen vom Impulsgenerator (10) angesteuerten Stimulationselektrode (4);
   - einer für die Verankerung im Ventrikel vorgesehenen Überwachungselektrode (6);
   - einer von dem Impulsgenerator (10) und von der Überwachungselektrode (6) angesteuerten elektronischen Zeitmeßschaltung (13;26;34) zur Messung des vom Belastungszustand des Herzens abhängigen Zeitintervalls $T_{AR}$ zwischen dem Stimulationsimpuls (A) im Atrium und der von der Überwachungselektrode (6) festgestellten diesem Stimulationsimpuls (A) zugeordneten Erregung (R) im Ventrikel, und
   - einer von der Zeitmeßschaltung (13;26;34) angesteuerten elektronischen Auswerteschaltung (18;28;30;36;38;45), durch die die Stimulationsfrequenz $F_{St}$

des Impulsgenerators (10) in Abhängigkeit von dem gemessenen Zeitintervall $T_{AR}$ zwischen einer einstellbaren Maximalfrequenz und einer einstellbaren Minimalfrequenz entsprechend einer linearen Beziehung $F_{st} = -a \cdot T_{AR} + b$, bei der a und b positive Konstanten darstellen, oder entsprechend einer s-förmig verlaufenden Beziehung geregelt wird.

2. Herzschrittmacher nach Anspruch 1, dadurch gekennzeichnet, daß die Auswerteschaltung (18;28;30;36;38;45) ein Verstellglied (19) umfaßt, durch das die Steilheit der linearen oder der s-förmigen Beziehung zwischen der Stimulationsfrequenz $F_{st}$ und dem gemessenen Zeitintervall $T_{AR}$, das heißt der Koeffizient a, patientenindividuell einstellbar ist.

3. Herzschrittmacher nach Anspruch 1 oder 2, dadurch gekennzeichnet, daß die Auswerteschaltung (18;28;30;36;38;45) ein Verstellglied (20) umfaßt, durch das der Koeffizient b der linearen Beziehung zwischen der Stimulationsfrequenz $F_{st}$ und dem gemessenen Zeitintervall $T_{AR}$ patientenindividuell einstellbar ist.

4. Herzschrittmacher nach einem oder mehreren der Ansprüche 1 bis 3, dadurch gekennzeichnet, daß die das Zeitintervall $T_{AR}$ zwischen dem Stimulationsimpuls und der von der Überwachungselektrode (6) festgestellten diesem Stimulationsimpuls zugeordneten Erregung im Ventrikel messende Zeitmeßschaltung aus einer Analogschaltung, insbesondere aus einem Spannungsintegrator (26), besteht.

5. Herzschrittmacher nach Anspruch 4, dadurch gekennzeichnet, daß die Auswerteschaltung aus einem von dem Spannungsintegrator (26) angesteuerten analogen Funktionsgenerator (28) und einem von diesem Funktionsgenerator (28) angesteuerten spannungsgesteuerten Monoflop (30) besteht.

6. Herzschrittmacher nach einem oder mehreren der Ansprüche 1 bis 5, dadurch gekennzeichnet, daß die das Zeitintervall $T_{AR}$ zwischen dem Stimulationsimpuls und der von der Überwachungselektrode (6) festgestellten diesem Stimulationsimpuls zugeordneten Erregung im Ventrikel messende Zeitmeßschaltung aus einer Digitalschaltung, insbesondere aus einem Zähler (34) besteht.

7. Herzschrittmacher nach Anspruch 6, dadurch gekennzeichnet, daß die Auswerteschaltung aus einem von dem Zähler (34) angesteuerten digitalen Funktionsgenerator (36) und einem von diesem Funktionsgenerator (36) angesteuerten Zähler (38) besteht.

8. Herzschrittmacher nach Anspruch 6, dadurch gekennzeichnet, daß die Auswerteschaltung einen Mikroprozessor (45) umfaßt.

9. Herzschrittmacher nach Anspruch 8, dadurch gekennzeichnet, daß der Mikroprozessor eine Regelschaltung zur Ermittlung und Regelung der dem jeweiligen Belastungsgrad entsprechenden optimalen Stimulationsfrequenz umfaßt, wobei durch diese Regelschaltung in bestimmten Zeitabständen die Stimulationsfrequenz jeweils so weit erhöht oder erniedrigt wird, daß die in der Zeitmeßschaltung gemessene $T_{AR}$-Zeit auf den jeweils niedrigsten Wert eingeregelt wird.

10. Herzschrittmacher nach einem oder mehreren der Ansprüche 1 bis 9, dadurch gekennzeichnet, daß die Steuerschaltung eine Erkennungsschaltung für intrakardiale, von der atrialen Stimulationselektrode (4) erfaßte Spontanaktionen umfaßt, wobei durch die Erkennungsschaltung bei Auftreten von Spontanaktionen (P) ein laufendes Zeitintervall ($T_{RA}$) abgebrochen und das sich an die Spontanaktion (P) anschließende Zeitintervall ($T_{PR}$) gemessen wird.

**Claims**

1. Frequency adaptible cardiac pacemaker with the following features:
   - a pulse generator (10);
   - a stimulation electrode (4) controlled by the pulse generator (10) and provided for anchoring in the atrium;
   - a monitoring electrode (6) provided for anchoring in the ventricle;
   - an electronic time measuring circuit (13; 26; 34), which is controlled by the pulse generator (10) and by the monitoring electrode (6), for the measuring of the time interval $T_{AR}$, which is dependent on the load state of the heart, between the stimulation pulse (A) in the atrium and the excitation (R), which is associated with this stimulation pulse (A) and established by the monitoring electrode (6), in the ventricle, and
   - an electronic evaluating circuit (18; 28; 30; 36; 38; 45), which is controlled by the time measuring circuit (13; 26; 34) and by which the stimulation frequency $F_{St}$ of the pulse generator (10) is regulated, in dependence on the measured time interval $T_{AR}$, between a settable maximum frequency and a settable minimum frequency in correspondence with a linear equation $F_{St} = -a \cdot T_{AR} + b$, in which a and b represent positive constants, or in correspondence with an equation with s-shaped course.

2. Cardiac pacemaker according to claim 1, characterised thereby that the evaluating circuit (18; 28; 30; 36; 38; 45) comprises an adjusting element (19), by which the steepness of the linear or s-shaped equation between the stimulation frequency $F_{St}$ and the measured time interval $T_{AR}$, that is to say the coefficient a, is settable for individual patients.

3. Cardiac pacemaker according to claim 1 or 2, characterised thereby that the evaluating circuit (18; 28; 30; 36; 38; 45) comprises an adjusting element (20), by which the coefficient b of the linear equation between the stimulation frequency $F_{St}$ and the measured time interval $T_{AR}$ is settable for individual patients.

4. Cardiac pacemaker according to one or several of claims 1 to 3, characterised thereby that the time measuring circuit, which measures the time interval $T_{AR}$ between the stimulation pulse and the excitation, in the ventricle, associated with this stimulation pulse and established by the monitoring electrode (6), consists of an analog circuit, particularly of a voltage integrator (26).

5. Cardiac pacemaker according to claim 4, characterised thereby that the evaluating circuit consists of an analog function generator (28) controlled by the voltage integrator (26) and a voltage-controlled monoflop (30) controlled by this function generator (28).

6. Cardiac pacemaker according to one or several or claims 1 to 5, characterised thereby that the time measuring circuit, which measures the time interval $T_{AR}$ between the stimulation pulse and the excitation, in the ventricle, associated with this stimulation pulse and established by the monitoring electrode (6), consists of a digital circuit, particularly of a counter (34).

7. Cardiac pacemaker according to claim 6, characterised thereby that the evaluating circuit consists of a digital function generator (36) controlled by the counter (34) and a counter (38) controlled by this function generator (36).

8. Cardiac pacemaker according to claim 6, characterised thereby that the evaluating circuit comprises a microprocessor (45).

9. Cardiac pacemaker according to claim 8, characterised thereby that the microprocessor comprises a regulating circuit for the ascertaining and regulation of the optimum stimulation frequency corresponding to the respective degree of loading, wherein the stimulation frequency is increased or decreased to such an extent each time at specific time intervals by this regulating circuit that the $T_{AR}$ time measured in the time measuring circuit is regulated to the respective lowest value.

10. Cardiac pacemaker according to one or several of claims 1 to 9, characterised thereby that the control circuit comprises a recognition circuit for intracardiac spontaneous activities detected by the atrial stimulation electrode (4), wherein on the occurrence of spontaneous activities (P) a time interval ($T_{RA}$) in progress is interrupted by the recognition circuit and the time interval ($T_{PR}$) adjoining the spontaneous activity (P) is measured.

**Revendications**

1. Stimulateur cardiaque s'adaptant à la fréquence, comportant les caractéristiques suivantes:
   - un générateur d'impulsions (10);
   - une électrode de stimulation (4), commandée par le générateur d'impulsions (10), prévue pour l'ancrage dans l'atrium;
   - une électrode de surveillance (6), prévue pour l'ancrage dans le ventricule ;
   - un circuit électronique pour la mesure du temps (13; 26; 34), commandé par le générateur d'impulsions (10) et par l'électrode de surveillance (6), pour la mesure de l'intervalle de temps $T_{AR}$ entre l'impulsion de stimulation (A) dans l'atrium, dépendant de l'état de charge du coeur, et l'excitation associée à cette impulsion de stimulation (A), constatée par l'électrode de surveillance (6), et
   - un circuit d'interprétation électronique (18; 28; 30; 36; 38; 45) commandé par le circuit de mesure du temps (13; 26; 34), par lequel la fréquence de stimulation $F_{St}$ du générateur d'impulsions (10) est réglée en fonction de l'intervalle de temps $T_{AR}$ mesuré entre une fréquence maximale réglable et une fréquence minimale réglable, de façon à correspondre à une relation linéaire

$$F_{St} = - a.T_{AR} + b,$$

a et b représentant des constantes positives,

ou de façon à correspondre à une relation en S.

2. Stimulateur cardiaque suivant la revendication 1, caractérisé en ce que le circuit d'interprétation (18; 28; 30; 36; 38; 45) comporte un organe de réglage (19), par lequel on peut régler, individuellement pour chaque patient, la pente de la relation, linéaire ou en S, entre la fréquence de stimulation $F_{St}$ et l'intervalle de temps $T_{AR}$ mesuré, c'est-à-dire le coefficient a.

3. Stimulateur cardiaque suivant la revendication 1 ou la revendication 2, caractérisé en ce que le circuit d'interprétation (18; 28; 30; 36; 38; 45) comporte un organe de réglage (20), par lequel on peut régler, individuellement pour chaque patient, le coefficient b de la relation linéaire entre la fréquence de stimulation $F_{St}$ et l'intervalle de temps $T_{AR}$ mesuré.

4. Stimulateur cardiaque suivant l'une quelconque ou plusieurs des revendications 1 à 3, caractérisé en ce que le circuit de mesure du temps, mesurant l'intervalle de temps $T_{AR}$ entre l'impulsion de stimulation et l'excitation dans le ventricule, associée à cette l'impulsion de stimulation et constatée par l'électrode de surveillance (6), est constitué par un circuit analogique, en particulier par un intégrateur de tension (26).

5. Stimulateur cardiaque suivant la revendication 4, caractérisé en ce que le circuit d'interprétation est constitué d'un générateur de fonction analogique (28), commandé par l'intégrateur de tension (26), et d'une bascule monostable (monoflop) (30) commandée par la tension, et commandée par ce générateur de fonction (28).

6. Stimulateur cardiaque suivant l'une quelconque ou plusieurs des revendications 1 à 5, caractérisé en ce que le circuit de mesure du temps, mesurant l'intervalle de temps $T_{AR}$ entre l'impulsion de stimulation et l'excitation dans le ventricule, associée à cette l'impulsion de stimulation et constatée par l'électrode de surveillance (6), est constitué par un circuit digital, en particulier par un compteur (34).

7. Stimulateur cardiaque suivant la revendication 6, caractérisé en ce que le circuit d'interprétation est constitué d'un générateur de fonction digitale (36) commandé par le compteur (34), et d'un compteur (38), commandé par ce générateur de fonction (36).

8. Stimulateur cardiaque suivant la revendication 6, caractérisé en ce que le circuit d'interprétation comporte un microprocesseur (45).

9. Stimulateur cardiaque suivant la revendication 8, caractérisé en ce que le microprocesseur (45) comporte un circuit de régulation pour émettre et réguler la fréquence de stimulation optimale correspondant à chaque degré de charge concerné, étant entendu que, par ce circuit de régulation, dans un intervalle de temps déterminé, la fréquence de stimulation est chaque fois augmentée ou diminuée assez pour que le temps $T_{AR}$, mesuré dans le circuit de mesure du temps, soit réglé sur la valeur la plus basse concernée.

10. Stimulateur cardiaque suivant l'une quelconque ou plusieurs des revendications 1 à 9, caractérisé en ce que le circuit d'interprétation comporte un circuit de reconnaissance pour des actions spontanées intracardiaques saisies par l'électrode de stimulation (4) de l'atrium, étant entendu que, par le circuit de reconnaissance, dans le cas d'apparition d'actions spontanées (P), l'intervalle de temps $T_{RA}$ en cours est interrompu et que l'intervalle de temps $T_{PR}$ faisant suite à l'action spontanée (P) est mesuré.

**Fig. 1**

**Fig. 2**

*Fig. 3*

*Fig. 4*

11

*Fig. 5*

*Fig. 6*

*Fig. 7*

*Fig. 8*